# EUROPEAN PATENT SPECIFICATION

(11) **EP 2 526 873 B1**
(45) Date of publication and mention of the grant of the patent: **28.08.2019**
(21) Application number: 12168851.9
(22) Date of filing: 22.05.2012
(51) Int. Cl.: A61B 17/00

(54) **Tissue dissectors**
Gewebedissektoren
Dissecteur de tissus

(30) Priority: 23.05.2011 US 201113113736
(43) Date of publication of application: 28.11.2012
(73) Proprietor: Covidien LP, Mansfield, MA 02048 (US)
(72) Inventor: Ladtkow, Casey M., Westminster, CO Colorado 80031 (US); Brannan, Joseph D., Erie, CO Colorado 80516 (US); Haley, Kaylen J., Westminster, CO Colorado 80031 (US); Willyard, Richard A., Longmont, CO Colorado 80501 (US)
(74) Representative: Soames, Candida Jane

(56) References cited:
- EP-A1- 1 719 451
- WO-A1-99/44520
- WO-A1-02/053221
- US-A1- 2009 230 167
- US-A1- 2010 057 087
- US-A1- 2010 217 151

## Description

### BACKGROUND

### Technical Field

The present disclosure relates to tissue dissectors and, more particularly, to deployable tissue dissectors that include a shaft having an expandable distal end.

### Background of Related Art

During an electrosurgical procedure, e.g., a thermal ablation procedure, target tissue is heated to high temperatures, i.e., temperatures high enough to ablate tissue. Under certain surgical environments, it is sometimes necessary to protect critical tissue structures, e.g., organ, bone matter, etc., adjacent the target tissue from the heat associated with the thermal ablation procedure. To protect adjacent or nearby tissue, the adjacent tissue is typically dissected, covered, shielded or otherwise treated. For example, one technique that is commonly utilized for protecting adjacent tissue structure during a thermal ablation procedure includes dissecting adjacent tissue by injecting a fluid, e.g., saline, CO2, D5W, etc., into a space between target tissue and the adjacent tissue. While this technique works well under certain surgical environments, this technique is limited, however, because it is difficult to control the location of the fluid and it is difficult to remove all the fluid from the body. In addition, and in the instance where the fluid is a gas, e.g., CO2, the CO2 often dissolves into the tissue, which requires the CO2 to be replenished (sometimes quite frequently) during a surgical procedure. As can be appreciated, having to replenish the CO2 during a surgical procedure may increase the length of time needed to effectively perform the surgical procedure.

US2010217151 A1 discloses a dissection device with an distal expandable section on the end of the introducer. The said expandable section is used to dissect a path for the end effector which accesses the surgical site via the introducer.

### SUMMARY

The invention is defined by claim 1 and the preferred embodiments are disclosed in the dependent claims. The present disclosure provides a tissue dissector. The tissue dissector includes an introducer that includes a lumen extending along a length thereof and defines a longitudinal axis therethrough. The introducer configured for placement adjacent to target tissue. A shaft operably coupled to the introducer is deployable from a distal end thereof and includes a proximal end for approximating the distal end of the shaft adjacent target tissue. The distal end of the shaft is movable from a non-expanded configuration to an expanded configuration for separating target tissue from neighboring tissue such that the neighboring tissue is not critically affected during the electrosurgical procedure. The distal end of the shaft may form a Faraday cage in the expanded configuration for stopping or impeding propagation of electrosurgical energy.

The present disclosure provides a system for electrosurgically treating tissue. The system includes a source of electrosurgical energy, an electrosurgical instrument that is adapted to operably couple to the source of electrosurgical energy and configured to electrosurgically treat tissue of interest and a tissue dissector. The tissue dissector includes an introducer that includes a lumen extending along a length thereof and defines a longitudinal axis therethrough. The introducer configured for placement adjacent to target tissue. A shaft is operably coupled to the introducer and is deployable from a distal end of the introducer. The shaft includes a proximal end for approximating the distal end of the shaft adjacent target tissue. The distal end of the shaft is movable from a non-expanded configuration to an expanded configuration for separating target tissue from neighboring tissue such that the neighboring tissue is not critically affected during the electrosurgical procedure.

The present disclosure also provides an exemplary method for electrosurgically treating tissue. A step of the method includes positioning an introducer of a tissue dissector adjacent target tissue. Deploying a shaft from the introducer between the target tissue and neighboring tissue is a step of the method. The method includes expanding a distal end of the shaft such that the neighboring tissue separates from the target tissue. And, electrosurgically treating the target tissue is a step of the method. The step of expanding a distal end may include the step of providing the distal end with a mesh configuration that is made from wire, wherein the wire is made from a material selected from the group consisting of shape memory alloy and elastomeric material. The step of deploying a shaft may include deploying a shaft including a plurality of slits defined along an outer periphery of the distal end of the shaft, the plurality of slits configured to facilitate transitioning of the distal end of the shaft from the non-expanded configuration to the expanded configuration. The step of expanding a distal end of the shaft may include the step of moving an actuator operably coupled to the distal end of the shaft, wherein the actuator is selected from the group consisting of a wire, cable and string.

### BRIEF DESCRIPTION OF THE DRAWINGS

Embodiments of the presently disclosed tissue dissectors are described hereinbelow with reference to the drawings wherein:
FIG. 1 is a schematic view of a system for performing an electrosurgical procedure according to an embodiment of the present disclosure;
FIGS. 2A-2B are schematic views of a tissue dissector associated with the system depicted in FIG. 1;
FIGS. 2C-2D are schematic views illustrating various distal end configurations that may be utilized with the tissue dissector depicted in FIGS. 2A and 2B;
FIGS. 3A-3D are schematic views of a tissue dissector configured for use with the system depicted in FIG. 1 according to another embodiment of the present disclosure;
FIG. 3E is a cross-sectional view taken along the line segment 3E in FIG. 3D;
FIGS. 4A-4B are schematic views of a tissue dissector configured for use with the system depicted in FIG. 1 according to yet another embodiment of the present disclosure; and
FIG. 5 is a top, elevational view of a shaft configured for use with the tissue dissectors depicted in FIGS. 2A, 3A and 4A.

### DETAILED DESCRIPTION OF THE EMBODIMENTS

Detailed embodiments of the present disclosure are disclosed herein; however, the disclosed embodiments are merely examples of the disclosure, which may be embodied in various forms. Therefore, specific structural and functional details disclosed herein are not to be interpreted as limiting, but merely as a basis for the claims and as a representative basis for teaching one skilled in the art to variously employ the present disclosure in virtually any appropriately detailed structure.

In the drawings and in the descriptions that follow, the term "proximal," as is traditional, will refer to an end of a surgical instrument that is closer to the user, while the term "distal" will refer to an end of a surgical instrument that is farther from the user.

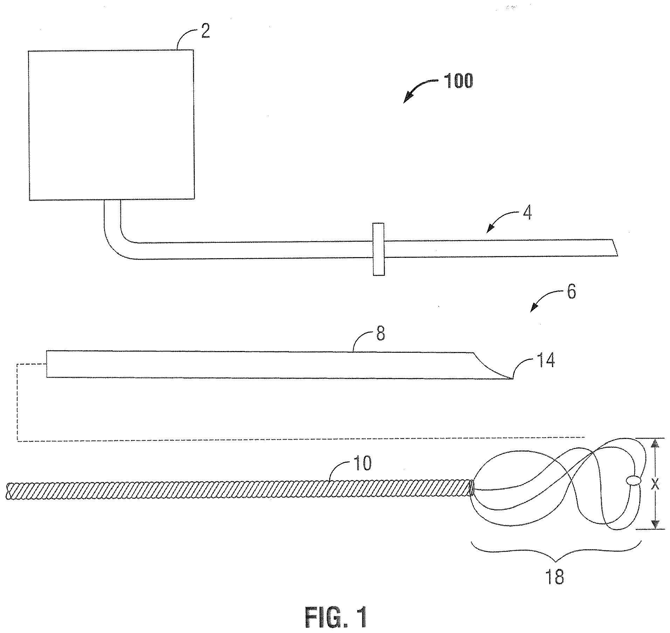

Referring to FIG. 1, a system 100 for electrosurgically treating tissue is illustrated including a source of electrosurgical energy, e.g., an electrosurgical generator 2, an electrosurgical instrument, e.g., a microwave antenna assembly 4, and a tissue dissector 6. The system 100 may be configured to perform one or more electrosurgical procedures for treating tissue including, but not limited to ablating, coagulating, and fulgurating tissue. For purposes herein, the system 100 is described in terms of a use for ablating tissue.

With continued reference to FIG. 1, electrosurgical generator 2 is configured to generate electrosurgical energy suitable for ablating tissue. Microwave antenna assembly 4 is adapted to operably couple to the electrosurgical generator 2 and is configured to electrosurgically treat tissue of interest (hereinafter referred to as target tissue "T"). For a more detailed description of the electrosurgical generator 4 including the microwave antenna assembly 4, reference is made to commonly-owned Patent Application Serial No. 12/606,767 to Brannan, published as US2010134887, filed on October 27, 2009.

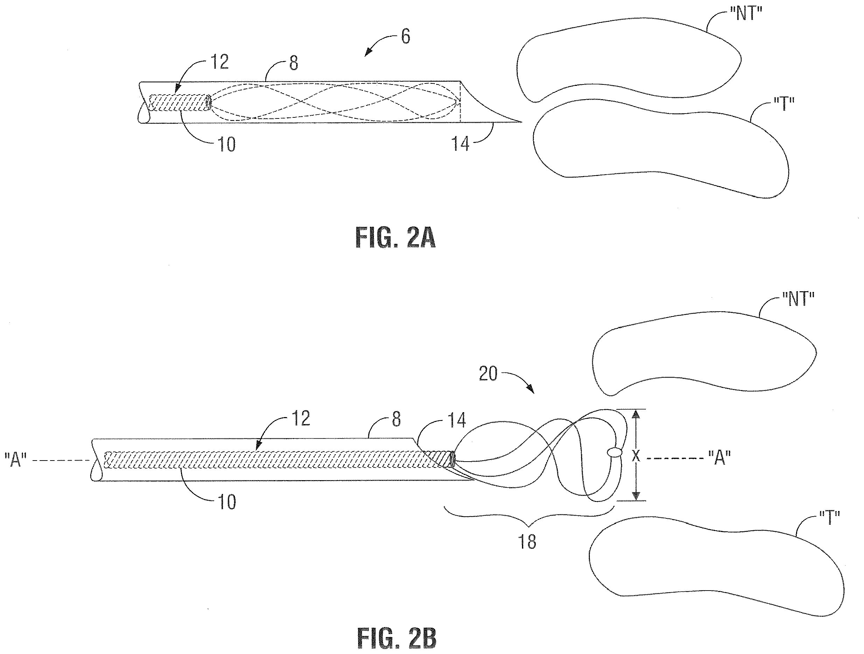

Continuing with reference to FIG. 1, and with reference to FIGS. 2A and 2B, an embodiment of the tissue dissector 6 is shown including an introducer (in the form of an introducer or catheter 8) and a shaft 10.

In the illustrated embodiment, catheter 8 is configured to pierce tissue and, subsequently, be positioned adjacent target tissue "T". To facilitate piercing tissue, the catheter 8 includes a generally sharpened distal tip 14 (FIGS. 1-2B). In certain embodiments, it may prove advantageous to provide the catheter 8 with a distal tip that is relative dull or blunt, such as, for example, in the instance where the catheter 8 is not configured to pierce tissue. Catheter 8 defines a longitudinal axis "A-A" therethrough and includes a lumen 12 defined therein that extends along a length thereof (FIGS. 2A-2B). The lumen 12 is configured to receive a shaft 10 therein (FIG. 2A) such that the shaft 10 or operable component associated therewith, e.g., a distal end 18, is deployable from an open distal end of the catheter 8 adjacent the distal tip 14 (FIG. 2B). Shaft 10 includes a proximal end (not shown) that is maneuverable by a user, e.g., a clinician, such that a user may position the shaft 10 within the lumen 12 of the catheter 8. Distal end 18 is movable from a non-expanded configuration (FIG. 2A) for loading the shaft 10 into, and deploying the distal end 18 from, the catheter 8, to an expanded configuration for separating neighboring tissue "NT" from target tissue "T" (FIG. 2B), described in greater detail below.

Distal end 18 operably couples to the shaft 10 by one or more suitable coupling methods, e.g., soldering, ultrasonic welding, etc.

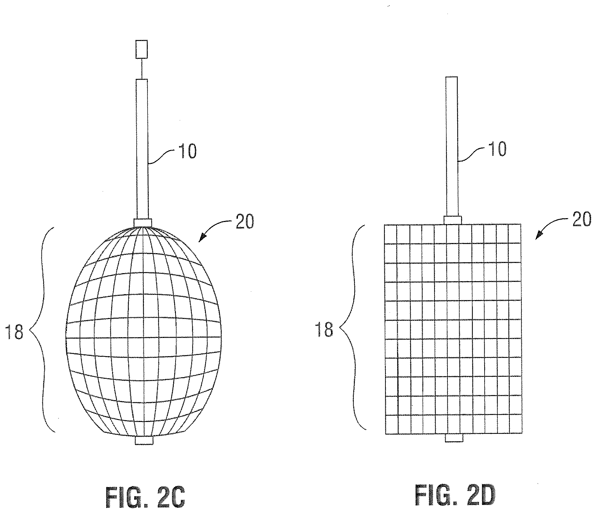

In the embodiment illustrated in FIGS. 2A and 2B, the distal end 18 of the shaft 10 includes a mesh structure configured from a plurality of wires 20. In some embodiments, the wires 20 are made from a material such as, for example, shape memory alloy, e.g., nitinol, and a compressible elastomeric material that is normally in an expanded configuration. In the expanded configuration, the distal end 18 of the shaft 10 may exhibit one or more suitable shapes. For example, in the expanded configuration the distal end 18 may include a shape including, but not limited to a sphere (FIG. 2C), a rectangle (FIG. 2D), and a helix (FIG. 2B). As can be appreciated, the specific shapes that the distal end 18 may exhibit in the expanded configuration may vary for a different surgical procedure, the type of tissue that is to be electrosurgically treated, the location of the tissue that is to be treated, a manufacturer's preference, etc.

Distal end 18 expands in a radial direction outward. As shown in FIG. 2B, in the expanded configuration, the helix of distal end 18 spans a distance (or includes a width) "x" that corresponds to a distance that the neighboring tissue "NT" is separated from the tissue of interest (FIG. 2B). This distance "x" is sufficient to ensure that the neighboring tissue "NT" is not critically affected during the electrosurgical procedure.

In certain instances, and in the expanded configuration, the distal end 18 of the shaft 10 may be configured to stop and/or impede the propagation of microwave energy during an ablation procedure. In this instance, it may prove advantageous to tightly weave the wires 20 of the distal end 18 such that the distal end 18 functions as a faraday cage, see FIGS. 2C and 2D for example.

Operation of the system 100 is now described in terms of use of a method for electrosurgically treating tissue. Catheter 8, initially, is utilized to pierce tissue such that the catheter 8 may be positioned adjacent target tissue "T", e.g., tissue that is to be electrosurgically treated (FIG. 2A). The shaft 10 is positioned within the lumen 12 of the catheter 8 and, subsequently, the distal end 18 is deployed from the catheter 8 such that the distal end 18 is positioned between the target tissue "T" and neighboring tissue "NT" (FIG. 2B). When the distal end 18 is deployed from the catheter 8, the distal end 18 transitions from the non-expanded configuration to an expanded configuration. As the distal end 18 transitions from the non-expanded configuration to the expanded configuration, the distal end 18 separates neighboring tissue "NT" from the target tissue "T". Thereafter, the target tissue "T" is electrosurgically treated via the microwave antenna assembly 4.

As can be appreciated, the tissue dissector 6 disclosed herein effectively separates and isolates the neighboring tissue "NT" from the target tissue "T" and reduces and/or eliminates the likelihood of the neighboring tissue "NT" being critically affected as the target tissue "T" is electrosurgically treated. This is accomplished without the need of having to introduce any extra fluid to the surgical environment, which, as noted above, may increase the length of time needed to effectively perform the surgical procedure.

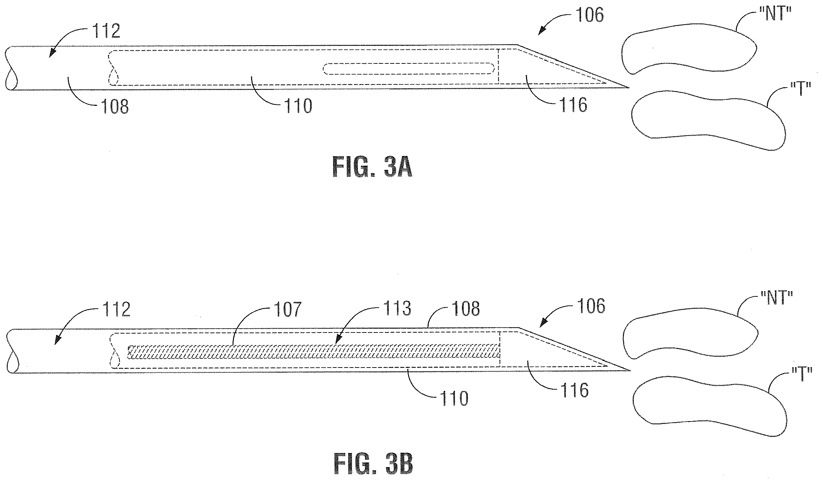

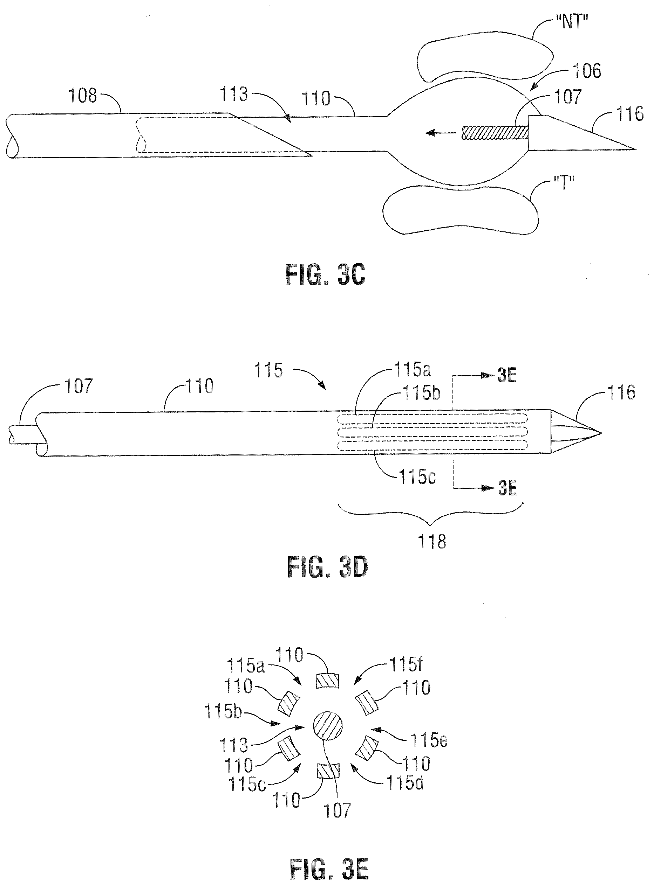

With reference to FIGS. 3A-3E, a tissue dissector according to another embodiment of the present disclosure is shown designated tissue dissector 106. Tissue dissector 106 is substantially similar to the tissue dissector 6. Accordingly, only those features that are unique to tissue dissector 106 are described in detail herein.

A cannula 108 is substantially similar to that of cannula 8. However, unlike cannula 8, cannula 108 is configured to receive a shaft 110 that, in the embodiment illustrated in FIGS. 3A-3E, is larger than a diameter of the shaft 10. The larger diameter of the shaft 110 is configured to accommodate an actuator 107, described in greater detail below.

Shaft 110 includes an elongated configuration having a generally circumferential shape when viewed in cross-section (FIG. 3E). Unlike shaft 10, a distal end 118 of shaft 110 includes a plurality of spaced slits or slots 115a-115f (collectively referred to as slits 115), as best seen in FIG. 3E. The slits 115 function to facilitate moving the distal end 118 from a non-expanded configuration (FIG. 3A) to an expanded configuration (FIG. 3C). That is, the slits 115 facilitate expansion and contraction of the distal end 118 of the shaft 110. In particular, the slits 115 allow the distal end 118 of the shaft 110 to "swell" or "bulge" about the slits 115 when the actuator 107 is pulled proximally.

In the embodiment illustrated in FIGS. 3A-3E, the six slits 115a-115f are evenly spaced at approximately 60 degrees apart from each other. However, the number of slits 115 may vary for a different surgical procedure, the type of tissue that is to be electrosurgically treated, the location of the tissue that is to be treated, a manufacturer's preference, etc. For example, in an instance where two (2) slits 115 are utilized, e.g., slits 115a and 115b, they be spaced approximately 180 degrees apart from each other; in an instance where three (3) slits 115 are utilized, e.g., slits 115a, 115b and 115c, they may be spaced approximately 120 degrees apart from each other; in an instance where four (4) slits 115 are utilized, e.g., slits 115a, 115b, 115c and 115d, they may be spaced approximately 90 degrees apart from each other; and in an instance where five (5) slits 115 are utilized, e.g., slits 115a, 115b, 115c, 115d and 115e, they may be spaced approximately 72 degrees apart from each other. One skilled in the art will appreciate that any number of slits may be utilized. Six (6) slits 115a-115f were found to provide an even distribution of an expansion force that is generated when the distal end 118 of the shaft 110 transitions from a non-expanded configuration, to an expanded configuration.

Unlike the shaft 10, shaft 110 includes a pointed or sharpened distal tip 116 (FIGS. 3A-3D) that is configured to pierce or penetrate tissue, e.g., target tissue "T" or neighboring tissue "NT," such that the distal end 118 may be temporarily anchored into target tissue "T" or neighboring tissue "NT," i.e., the sharpened distal tip 116 is configured to pierce tissue such that a portion of the distal tip 116 may secure to the tissue. As can be appreciated, temporarily anchoring the distal tip 116 into target tissue "T" or neighboring tissue "NT" may facilitate pulling or "drawing" back the catheter 108 during deployment of the distal end 118 from the distal end of the catheter 8.

The actuator 107 extends through a lumen 113 of the shaft 110 and operably couples to the distal tip 116 adjacent the distal end 118 of the shaft 110, as best seen in FIGS. 3B and 3C. The actuator 107 is configured to move the distal end 118 of the shaft 110 from the non-expanded configuration, to the expanded configuration when the actuator 107 is pulled proximally. To this end, the actuator 107 may be any suitable actuator 107 including but not limited to a wire, cable and string. In the illustrated embodiment, the actuator 107 is in a form of a cable.

In use, catheter 108, initially, is utilized to pierce tissue such that the catheter 108 may be positioned adjacent target tissue "T" (FIG. 3A). The shaft 110 is positioned within the lumen 112 of the catheter 108 and, subsequently, the distal end 118 is deployed from the catheter 108 such that the distal end 118 is positioned between the target tissue "T" and neighboring tissue "NT" (FIG. 3C). When the distal end 118 is deployed from the catheter 108 and positioned between the neighboring tissue "NT" and target tissue "T," the actuator 107 is actuated, e.g., pulled proximally, which, in turn, causes the slits 115 of the distal end 118 to move or transition from the initial non-expanded configuration, to the expanded configuration. As the distal end 118 transitions from the non-expanded configuration to the expanded configuration, the distal end 118 separates the neighboring tissue "NT" from the target tissue "T". Thereafter, the target tissue "T" is electrosurgically treated as described above.

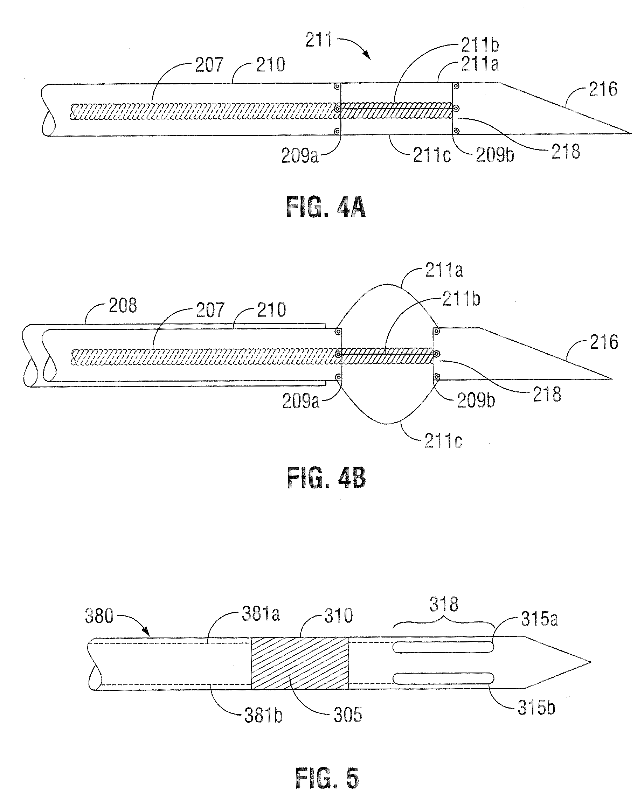

With reference to FIGS. 4A and 4B, a tissue dissector according to another embodiment of the present disclosure is shown and designated tissue dissector 206. Tissue dissector 206 is substantially similar to the tissue dissector 106. Accordingly, only those features that are unique to tissue dissector 206 are described in detail herein.

A shaft 210 includes a first ring 209a and second ring 209b that are operably disposed at a distal end 218 of the shaft 210 and are coupled to one another via one or more spaced-apart resilient members 211 (three (3) resilient members 211a-211c are shown in the figures) that extend along the longitudinal axis "A-A." The first and second rings 209a and 209b are configured to couple the distal end 218 of the shaft 210 to a distal tip 216 thereof. The rings 209a and 209b including the resilient members 211a-211c function similar to that of slits 115. That is, the rings 209a and 209b including the resilient members 211a-211c facilitate moving the distal end 218 of the shaft 210 from the non-expanded configuration to the expanded configuration. The resilient members 211a-211c may be made from any suitable resilient materials including but not limited to a wire, a band, a spring, etc. In the embodiment illustrated in FIGS. 4A and 4B, the resilient members 211a-211c are wire strips that are bent around (or otherwise coupled to) the rings 209a and 209b.

In use, a catheter 208 (FIG. 4B), initially, is utilized to pierce tissue such that the catheter 208 may be positioned adjacent target tissue "T. The shaft 210 is positioned within a lumen (not explicitly shown) of the catheter 208 and, subsequently, the distal end 218 is deployed from the catheter 208 such that the distal end 218 is positioned between the target tissue "T" and neighboring tissue "NT." When the distal end 218 is deployed from the catheter 208 and positioned between the neighboring tissue "NT" and target tissue "T," a cable 207, is pulled proximally, which, in turn, causes the resilient members 211a-211c of the distal end 218 to move or transition from a non-expanded configuration, to the expanded configuration. As the distal end 218 transitions from the non-expanded configuration to the expanded configuration, the distal end 218 separates the neighboring tissue "NT" from the target tissue "T". Thereafter, the target tissue "T" is electrosurgically treated.

From the foregoing, and with reference to the various figure drawings, those skilled in the art will appreciate that certain modifications can also be made to the present disclosure without departing from the scope of the same. For example, it is contemplated that one or more guide wires 380 may operably couple by one or more suitable coupling methods to a shaft 310 that is configured for use with any of the aforementioned tissue dissectors (FIG. 5). The guide wires 380 function as a steering mechanism and are configured to move or steer the shaft 310. More specifically, two independently controllable guide wires 381a and 381b may be operably coupled to the shaft 310 and spaced 180 degrees apart from each other. Separating the guide wires 381a and 381 180 degrees apart from one another provides an even distribution of a pull force across the shaft 310. In the embodiment illustrated in FIG. 5, the guide wires 381a and 381b are operably-disposed within corresponding grooves (not explicitly shown) along an outer periphery of the shaft 310. The guide wires 381a and 381b couple adjacent to the distal end 318 of the shaft 310 by one or more suitable coupling methods, e.g., soldering. For illustrative purposes, distal end 318 includes two slots 315a and 315b spaced approximately 180 degrees apart from each other.

The guide wires 381a and 381b are configured such that actuating, e.g., pulling, a respective one of the guide wires 381a and 381b causes the shaft 310 including a distal end 318 to move laterally or transversely across the longitudinal axis "A-A" in a respective direction, e.g., left or right. Utilizing the guide wires 381a and 381b facilitates positioning the distal end 318 of the shaft 310 adjacent the target tissue "T" and/or neighboring tissue "NT". For illustrative purposes, when the guide wire 381a is pulled, the shaft 310 including the distal end 318 moves to the left and when the guide wire 381b is pulled, the shaft 310 including the distal end 318 moves to the right.

A portion 305 of the shaft 310 is configured to articulate when either of the guide wires 381a and 381b is pulled. To this end, the portion 305 may include one or more links that are configured to facilitate articulation. The portion 305 of the shaft 310 (or the shaft 310 itself) may be substantially resilient to facilitate bending in one or more directions or the portion 305 of the shaft 310 (or the shaft 310 itself) may be malleable. In the embodiment illustrated in FIG. 5, portion 305 is made from a material that is malleable, e.g., a relatively pliable or compliant plastic, and configured such that when either of the guide wires 381a and 381b is pulled, the shaft 310 bends or moves about the portion 305, which, in turn, steers or moves the distal end 318 in a corresponding direction. The malleable portion 305 is configured to maintain the distal end 318 in the corresponding direction until either one of the guide wires 381 or 381b is actuated. Thus, inadvertent contact between target tissue "T", neighboring tissue "NT" or other tissue structure and the distal end 318 does not cause the distal end 318 to move.

As can be appreciated, the number of guide wires (and or the location of them along the periphery of the shaft 310) may vary for a different surgical procedure, the type of tissue that is to be electrosurgically treated, the location of the tissue that is to be treated, a manufacturer's preference, etc. For example, in one particular embodiment, four (4) guide wires may be operably disposed along the periphery of the shaft 310. In this instance, the four (4) guide wires may be spaced-apart at 90 degree intervals from each other and configured to move the shaft 310 in a corresponding direction, e.g., left and right of the longitudinal axis "A-A" and above and below the longitudinal axis "A-A."

Use of any of the aforementioned tissue dissectors, e.g., tissue dissector 206, with a shaft 310 including guide wires 381a and 381b is substantially similar as that described above. One difference being, the guide wires 381a and 381b may be utilized to move or "steer" shaft 310 including the distal end 318, prior to or after the distal end 318 is moved to the expanded condition. As can be appreciated, having the capability of "steering" the distal end 318 may provide an end user with a significant mechanical advantage, especially in the instance where target tissue is in a compromised or hard to reach location.

While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope of the claims appended hereto.

## Claims

1. A system (100) for electrosurgically treating target tissue, comprising:
a source of electrosurgical energy (2);
an electrosurgical instrument (4) adapted to operably couple to the source of electrosurgical energy and configured to electrosurgically treat target tissue;
a tissue dissector (6, 106), comprising:
an introducer (8) including a lumen extending along a length thereof and defining a longitudinal axis therethrough, the introducer configured for placement adjacent target tissue; and
a shaft (10, 110, 210, 310) operably coupled to the introducer and deployable from a distal end thereof, the introducer including a proximal end for approximating the distal end of the shaft adjacent target tissue, the distal end of the shaft movable from a non-extended, non-expanded configuration to an expanded configuration for separating target tissue from neighbouring tissue, wherein in the expanded configuration the distal end of the shaft spans a distance sufficient such that neighbouring tissue is not critically affected during the electrosurgical procedure.

2. A system according to claim 1, wherein the distal end expands in a radial direction.

3. A system according to claim 1 or 2, wherein the distal end of the shaft includes a shape selected from the group consisting of a sphere, a rectangle, and a helix when in the expanded configuration.

4. A system according to claim 1, 2 or 3 wherein at least two independently controllable guide wires (381a, 381b) are operably coupled to the shaft, each of the at least two independently controllable guide wires configured such that upon actuation of a respective one of the at least two independently controllable guide wires moves the shaft including the distal end transversely relative the longitudinal axis, preferably wherein the at least two guide wires are operably disposed along an outer periphery of the shaft and are spaced 180 degrees apart from one another.

5. A system according to claim 4, wherein a portion of the shaft is malleable to facilitate transverse movement of the shaft relative to the longitudinal axis, wherein the malleable portion of the shaft maintains the shaft including the distal end thereof in the transverse position until a respective one of the at least two guide wires is actuated.

6. A system according to any one of claims 1 to 5, wherein the distal end includes a mesh configuration that is made from wire.

7. A system according to claim 6, wherein the wire is made from a material selected from the group consisting of shape memory alloy and elastomeric material.

8. A system according to any one of claims 1 to 5, wherein a plurality of slits (115a-f) is defined along an outer periphery of the distal end of the shaft, preferably wherein the plurality of elongated slits is further defined by six elongated slits that are spaced approximately 60 degrees apart from each other.

9. A system according to any one of claims 1 to 5, wherein first and second rings (209a, 209b) are operably disposed at the distal end of the shaft and are coupled to one another via at least two spaced-apart resilient members that extend along the longitudinal axis, the first and second rings configured to couple to the distal end of the shaft and configured to facilitate moving the distal end of the shaft from the non-expanded configuration to the expanded configuration.

10. A system according to claim 9, wherein the at least two resilient members are one of a wire and a band.

11. A system according to claim 1, 8, 9 or 10 further comprising:
an actuator (107, 207) extending through a lumen defined in the shaft, the actuator operably coupled to the distal end and configured to transition the distal end of the shaft from the non-expanded configuration to the expanded configuration when the actuator is actuated.

12. A system according to claim 11, wherein the actuator is selected from the group consisting of a wire, cable and string.

13. A system according to claim 11 or 12, wherein the actuator operably couples to a distal tip of the distal end of the shaft.

14. A system according to any preceding claim, the shaft comprising a pointed or sharpened distal tip (116,216) for piercing or penetrating tissue.

## Patentansprüche

1. System (100) zur elektrochirurgischen Behandlung von Zielgewebe, umfassend:
eine Quelle elektrochirurgischer Energie (2);
ein elektrochirurgisches Instrument (4), das zum betriebsmäßigen Koppeln mit der Quelle elektrochirurgischer Energie angepasst und zum elektro-chirurgischen Behandeln von Zielgewebe konfiguriert ist;
einen Gewebedissektor (6, 106), umfassend:
eine Einführvorrichtung (8), die ein Lumen aufweist, das sich entlang einer Länge davon erstreckt und eine Längsachse dort hindurch definiert, wobei die Einführvorrichtung zur Platzierung benachbart zu Zielgewebe konfiguriert ist; und
einen Schaft (10, 110, 210, 310), der mit der Einführvorrichtung betriebsmäßig gekoppelt und von einem distalen Ende derselben aus einsetzbar ist, wobei die Einführvorrichtung ein proximales Ende zum Annähern des distalen Endes des Schafts benachbart zu Zielgewebe aufweist, wobei das distale Ende des Schafts von einer nicht verlängerten, nicht ausgedehnten Konfiguration zu einer ausgedehnten Konfiguration beweglich ist, um Zielgewebe von benachbartem Gewebe zu trennen, wobei in der ausgedehnten Konfiguration das distale Ende des Schafts einen Abstand überspannt, der ausreichend ist, so dass benachbartes Gewebe während des elektrochirurgischen Eingriffs nicht kritisch beeinflusst wird.

2. System nach Anspruch 1, wobei sich das distale Ende in radialer Richtung ausdehnt.

3. System nach Anspruch 1 oder 2, wobei das distale Ende des Schafts eine Form aufweist, die ausgewählt ist aus der Gruppe, bestehend aus einer Kugel, einem Rechteck und einer Wendel, wenn es sich in der ausgedehnten Konfiguration befindet.

4. System nach Anspruch 1, 2 oder 3, wobei mindestens zwei unabhängig steuerbare Führungsdrähte (381a, 381b) betriebsmäßig mit dem Schaft gekoppelt sind, wobei jeder der mindestens zwei unabhängig steuerbaren Führungsdrähte konfiguriert ist, so dass er bei Betätigung eines entsprechenden der mindestens zwei unabhängig steuerbaren Führungsdrähte den Schaft einschließlich des distalen Endes quer relativ zu der Längsachse bewegt, wobei die mindestens zwei Führungsdrähte vorzugsweise betriebsmäßig entlang eines äußeren Umfangs des Schafts angeordnet und 180 Grad voneinander beabstandet sind.

5. System nach Anspruch 4, wobei ein Abschnitt des Schafts formbar ist, um eine Querbewegung des Schafts relativ zu der Längsachse zu ermöglichen, wobei der formbare Abschnitt des Schafts den Schaft einschließlich seines distalen Endes in einer Querposition hält, bis ein entsprechender der mindestens zwei Führungsdrähte betätigt wird.

6. System nach einem der Ansprüche 1 bis 5, wobei das distale Ende eine Netzkonfiguration aufweist, die aus Draht hergestellt ist.

7. System nach Anspruch 6, wobei der Draht aus einem Material hergestellt ist, das ausgewählt ist aus der Gruppe, bestehend aus einem Formgedächtnismaterial und einem elastomeren Material.

8. System nach einem der Ansprüche 1 bis 5, wobei eine Vielzahl von Schlitzen (115a-f) entlang eines äußeren Umfangs des distalen Endes des Schaft definiert ist, wobei die Vielzahl von länglichen Schlitzen vorzugsweise weiter durch sechs längliche Schlitze definiert ist, die etwa 60 Grad voneinander beabstandet sind.

9. System nach einem der Ansprüche 1 bis 5, wobei erste und zweite Ringe (209a, 209b) betriebsmäßig an dem distalen Ende des Schafts angeordnet sind und über mindestens zwei beabstandete elastische Elemente, die sich entlang der Längsachse erstrecken, miteinander gekoppelt sind, wobei die ersten und zweiten Ringe konfiguriert sind, um sich mit dem distalen Ende des Schafts zu koppeln, und konfiguriert sind, um eine Bewegung des distalen Endes des Schafts von der nicht ausgedehnten Konfiguration zu der ausgedehnten Konfiguration zu ermöglichen.

10. System nach Anspruch 9, wobei die mindestens zwei elastischen Elemente eines von einem Draht und einem Band sind.

11. System nach Anspruch 1, 8, 9 oder 10, weiter umfassend:
einen Aktuator (107, 207), der sich durch ein Lumen erstreckt, das in dem Schaft definiert ist, wobei der Aktuator mit dem distalen Ende betriebsmäßig gekoppelt und konfiguriert ist, um das distale Ende des Schafts von der nicht ausgedehnten Konfiguration in die ausgedehnten Konfiguration zu überführen, wenn der Aktuator betätigt wird.

12. System nach Anspruch 11, wobei der Aktuator ausgewählt ist aus der Gruppe, bestehend aus einem Draht, einem Kabel und einer Schnur.

13. System nach Anspruch 11 oder 12, wobei sich der Aktuator betriebsmäßig mit einer distalen Spitze des distalen Endes des Schafts koppelt.

14. System nach einem vorstehenden Anspruch, wobei der Schaft eine spitze oder geschärfte distale Spitze (116, 216) zum Durchstechen oder Eindringen in Gewebe umfasst.

## Revendications

1. Système (100) pour un traitement électrochirurgical d'un tissu cible, comprenant :
une source d'énergie électrochirurgicale (2) ;
un instrument électrochirurgical (4) adapté pour se coupler fonctionnellement à la source d'énergie électrochirurgicale et configuré pour traiter électrochirurgicalement un tissu cible ;
un dissecteur de tissu (6, 106), comprenant :
un introducteur (8) incluant une lumière s'étendant le long d'une longueur de celui-ci et définissant un axe longitudinal à travers celui-ci, l'introducteur étant configuré pour un placement adjacent à un tissu cible ; et
une tige (10, 110, 210, 310) couplée fonctionnellement à l'introducteur et déployable à partir d'une extrémité distale de celle-ci, l'introducteur incluant une extrémité proximale pour une approximation de l'extrémité distale de la tige adjacente à un tissu cible, l'extrémité distale de la tige étant mobile d'une configuration non étendue, non étirée à une configuration étirée pour une séparation de tissu cible d'un tissu voisin, dans lequel dans la configuration étirée, l'extrémité distale de la tige couvre une distance suffisante de sorte qu'un tissu voisin n'est pas affecté de manière critique pendant la procédure électrochirurgicale.

2. Système selon la revendication 1, dans lequel l'extrémité distale s'étire dans une direction radiale.

3. Système selon la revendication 1 ou 2, dans lequel l'extrémité distale de la tige inclut une forme sélectionnée dans le groupe constitué d'une sphère, d'un rectangle, et d'une hélice lorsqu'elle est dans la configuration étirée.

4. Système selon la revendication 1, 2 ou 3 dans lequel au moins deux fils de guidage (381a, 381b) pouvant être commandés de manière indépendante sont couplés fonctionnellement à la tige, chacun des au moins deux fils de guidage pouvant être commandés de manière indépendante étant configuré de sorte que lors d'un actionnement d'un fil respectif parmi les au moins deux fils de guidage pouvant être commandés de manière indépendante, la tige incluant l'extrémité distale se déplace transversalement par rapport à l'axe longitudinal, de préférence dans lequel les au moins deux fils de guidage sont disposés fonctionnellement le long d'une périphérie extérieure de la tige et sont espacés de 180 degrés l'un de l'autre.

5. Système selon la revendication 4, dans lequel une partie de la tige est malléable pour faciliter un mouvement transversal de la tige par rapport à l'axe longitudinal, dans lequel la partie malléable de la tige maintient la tige incluant l'extrémité distale de celle-ci dans la position transversale jusqu'à ce qu'un fil respectif des au moins deux fils de guidage soit actionné.

6. Système selon l'une quelconque des revendications 1 à 5, dans lequel l'extrémité distale inclut une configuration en treillis qui est réalisée en fil.

7. Système selon la revendication 6, dans lequel le fil est réalisé en un matériau sélectionné dans le groupe constitué d'alliage à mémoire de forme et de matériau élastomère.

8. Système selon l'une quelconque des revendications 1 à 5, dans lequel une pluralité de fentes (115a-f) est définie le long d'une périphérie extérieure de l'extrémité distale de la tige, de préférence dans lequel la pluralité de fentes allongées est définie en outre par six fentes allongées qui sont espacées d'environ 60 degrés l'une de l'autre.

9. Système selon l'une quelconque des revendications 1 à 5, dans lequel des première et deuxième bagues (209a, 209b) sont disposées fonctionnellement à l'extrémité distale de la tige et sont couplées l'une à l'autre via au moins deux éléments résilients espacés qui s'étendent le long de l'axe longitudinal, les première et deuxième bagues étant configurées pour se coupler à l'extrémité distale de la tige et configurées pour faciliter un mouvement de l'extrémité distale de la tige de la configuration non étirée à la configuration étirée.

10. Système selon la revendication 9, dans lequel les au moins deux éléments résilients sont l'un parmi un fil et une bande.

11. Système selon la revendication 1, 8, 9 ou 10 comprenant en outre :
un actionneur (107, 207) s'étendant à travers une lumière définie dans la tige, l'actionneur étant couplé fonctionnellement à l'extrémité distale et configuré pour une transition de l'extrémité distale de la tige de la configuration non étirée à la configuration étirée lorsque l'actionneur est actionné.

12. Système selon la revendication 11, dans lequel l'actionneur est sélectionné dans le groupe constitué d'un fil, d'un câble et d'une chaîne.

13. Système selon la revendication 11 ou 12, dans lequel l'actionneur se couple fonctionnellement à une pointe distale de l'extrémité distale de la tige.

14. Système selon l'une quelconque des revendications précédentes, la tige comprenant une pointe distale (116, 216) pointue ou aiguisée pour un perçage ou une pénétration d'un tissu.
